# EUROPEAN PATENT APPLICATION

(11) **EP 2 022 413 A1**
(43) Date of publication of application: **11.02.2009**
(21) Application number: 07252991.0
(22) Date of filing: 30.07.2007
(51) Int. Cl.: A61B 17/12

(54) **Medical device for occlusion of blood flow**

(71) Applicant: Vasmo, Inc., Indianapolis IN 46218 (US)
(72) Inventor: Mohanraj, Subram, Fishers, Indiana 46038 (US)
(74) Representative: Atkinson, Peter Birch

(57) **Abstract**

A device, kit and method are disclosed, including magnetic microparticles, which can be administered into a blood vessel or fluid vessel, and an external magnetic field source usable to selectively form an occlusion or blockade in the fluid vessel such as a blood vessel.

## Description

### Background of the Invention

Vascular blood flow is now most commonly occluded during surgery by either clamping or ligation. Blood vessels are lined internally with a single layer of endothelial cells which are joined laterally by tight junctions and which overlie a layer of smooth muscle cells. This endothelial lining forms a permeability barrier to prevent the passage of large molecules between the blood and the extra corporeal space. Clamping or ligation results in trauma to the vessel and disruption of the endothelial cell permeability barrier (Barone GW, Conerly JM, Farley PC, Flanagan TL, Kron IL, Surgery 105:465-471, 1989; Moore WM, Manship LL, Bunt TJ, Am. Surg. 5:392-400, 1985; Margovsky Al, Lord RSA, Chambers AJ, Aust. N. Z. J. Surg. 67:448-451, 1997). In one study, these effects persisted for at least two weeks after a clamping period of only five minutes (Jackiewicz TA, McGeachie JK, Tennant M, Microsurg. 17:674-680, 1996). Compromising the permeability barrier allows the passage of factors in the blood which promote smooth muscle cell growth such as the platelet-derived growth factor (Jackiewicz TA, McGeachie JK, Tennant M, Microsurg. 17:674-680, 1996). Clamp injury can therefore cause later formation of atherosclerotic plaque and stenosis at the clamp site (Margovsky Al, Lord RSA, Chambers AJ, Aust. N. Z. J. Surg. 67:448-451, 1997). In addition, clamping can cause immediate peri-operative complications such as arterial dissection (Litchford B, Okies JE, Sugimura S, Starr A, J. Thorac. Cardiovasc. Surg. 72:709-713, 1976) or thrombosis due to embolization of atherosclerotic plaque to distal portions of the circulation (Archie JP Jr, Am. Surg. 54:167-168, 1988). Diseased vessels, such as those encountered during coronary artery revascularization, are especially prone to both immediate and long-term deleterious effects of clamping or ligation (Manship LL, Moore WM, Bynoe R, Bunt TJ, Am. Surg. 5:401-406, 1985). Noncompressible hemorrhage continues to be a primary cause of death in both military and civilian trauma (Bellamy R F. Mil. Med. 149:55-62, 1984; Sauaia A et al J. Trauma 38:185-193, 1995). Currently there is no treatment short of surgery for severe abdominal bleeding resulting from either blunt or penetrating injury. Vascular injuries in the region of the groin continue to be largely untreatable.

The use of magnetic particles for different applications in medicine has been reported for a long time. The most representative examples follow.
U.S. Patents 4,247,406 (Widder et al, 1981), 4,345,588 (Widder et al, 1982), 4,331,654 (Morris et al, 1982), 4,501,726 (Schroder et al, 1985), 4,690,130 (Mirell SG, 1987), 5,411,730 (Kirpotin et al, 1995), 5,427,767 (Kresse et al, 1995), 5,753,477 (Chan, 1998), and U.S. Patent Application 0219785 A1 (Hallahan et al., 2003) disclose the use of magnetic carriers for localized delivery of therapeutic or diagnostic agents. U.S. Patent 5,314,679 (Lewis et al, 1994) and U.S. Patent Application 09/852,421 (Unger, 2003) disclose the use of magnetic particles as contrast agents for magnetic resonance imaging. U.S. Patent Application 10/389,708 (Schwartz et al, 2003) discloses a method to embolize a vascular site using a microparticle comprised of a hydrolyzable crosslinked hydrogel. In this patent application, magnetic particles are also included as contrast agents in the hydrogel for magnetic resonance imaging. The mechanism of embolization here is the hydrolyzation of the crosslinked hydrogel. U.S. Patent 6,303,487 B1 (Consigny PM, 2001) discloses the use of magnetic particles in the focal delivery of cells.

U.S. Patent 6,364,823 B1(Garibaldi et al, 2002) discloses the use of magnetic particles for controlling the delivery of an embolic agent to a vascular defect. The purpose of Garibaldi's work is the delivery of embolic materials to the site of the vascular defect using magnetic means. A liquid embolic agent is provided with a magnetic constituent to be drawn into the defect using an applied magnetic field. The embolic agent, after reaching the vascular defect, precipitates a polymer which in combination with a glue, seals the vascular defect. In essence, the purpose of the magnetic material is to deliver the embolic agent with the precipitating polymer and glue to the site of the vascular defect. This application is similar to the targeted delivery of pharmaceutical agents to a particular site except that the agent here is an embolic material. Basically, in the above applications, magnetic particles have been used as a carrier to take a pharmaceutical or embolic agent to a particular location.

U.S. Patent 5,236,410 (Granov et al, 1993) discloses a method of treatment for a tumor using a magnetically hard, radio-opaque, ferromagnetic material suspended in an oil solution of an oil-soluble anti-tumor substance. A suspension of demagnetized ferromagnetic particles in an oil solution of an anti-tumor substance is drawn into the area of the tumor using an applied magnetic field. The ferromagnetic particles become remagnetized and, because of the high residual magnetism of the hard ferromagnetic material, form a porous body of aggregates at the tumor area. One purpose of the oil-suspended hard ferromagnetic material is to deliver as a carrier the oil-soluble anti-tumor substance to the tumor area and another purpose is to retain it within the tumor area by embolization of the hard ferromagnetic material. The major purpose of the oil-suspended hard ferromagnetic material, however, is for its use in hyperthermia. The distinction of Granov's method, compared to other hyperthermia methods or chemotherapy, is that Granov utilizes both chemotherapy and hyperthermia in trying to produce necrosis of the tumor tissue. U.S. Patent 6,149,576 (Gray et al, 2000) discloses the use of a ferromagnetic material to treat a tumor tissue by producing hyperthermia with the application of a rotational magnetic field.

U.S. Patent 4,364,377 (Smith FW, 1982) discloses a method to patch a bleeding lesion of a blood vessel from the outside of the blood vessel in the gastrointestinal tract. Under his method a pre-made tamponading mass ("mud", cream) having ferromagnetic properties was introduced into a large body cavity (GI tract) near the bleeding lesion (outside the blood vessel) using a piston and cylinder. An external magnetic field generator was used to move the pre-made tamponading mass into position and to cover the lesion hole. In Smith's method, the tamponading mass was applied to the outside of the bleeding vessel and patch work took place outside the blood vessel.

The principal embodiment of the present invention will occlude blood flow in a blood vessel during elective or emergency surgical procedures. Presently vascular blood flow in a blood vessel is most commonly occluded during surgery by either clamping or ligation. Traditional clamping or ligation results in trauma to the vessel and disruption of the endothelial cell permeability barrier as described above. The present invention is a better clamping method or device to occlude vascular blood flow in a blood vessel during surgery.

### Summary of the Invention

The present invention discloses a method for the occlusion of blood vessels or fluid vessels to stop or control the flow of blood or fluid during elective or emergency surgical procedures or following traumatic injury. The present invention also discloses a medical device, kit, or tool for occluding or controlling blood flow or fluid flow. In this application of the invention, a plug or blockade is formed within the blood vessel or fluid vessel to occlude or control blood flow or fluid flow when (a) a dispersion of magnetic microparticles is introduced into a blood vessel or fluid vessel, and (b) an external magnetic field source is placed or held at the desired site near or above the path of the bloodstream or fluid flow. The method or device can occlude blood flow or fluid flow in a reversible or non-reversible manner for a variety of surgical, medical, and other applications.

### Brief Description of the Drawings - Figures

Figures 1-13 are angiograms showing vessels during different time periods in the method:
Figures 1-3: Evaluation of MMD on the right carotid artery of a farm pig.
   Fig. 1: A baseline angiogram of a pig's right carotid artery.
   Fig. 2: An angiogram with an external magnetic field source placed on the skin above the pig's right carotid artery.
   Fig. 3: An angiogram of the pig's occluded right carotid artery after microparticle infusion.
Figures 4-6: Evaluation of MMD on the superficial femoral artery of a farm pig.

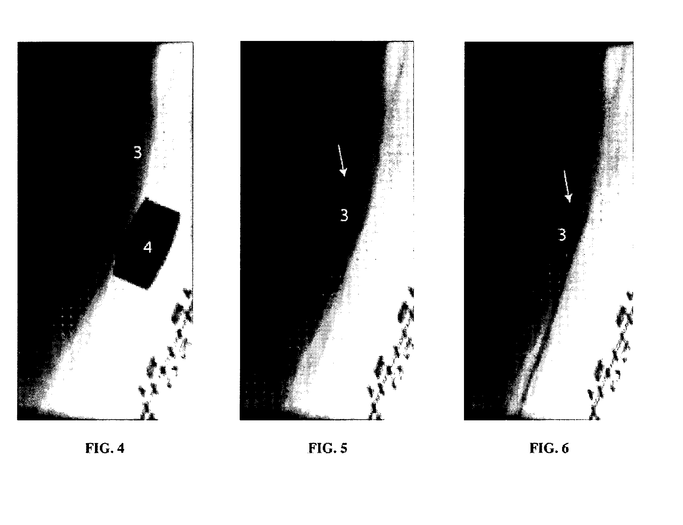

   Fig 4: Selective total occlusion of one of the superficial femoral arteries of a farm pig is shown after less than one minute of microparticle infusion.
   Fig. 5: Partial resumption of blood flow is shown after three minutes of removal of the external magnetic field source.
   Fig. 6: Complete resumption of the blood flow is shown after eight minutes of removal of the external magnetic field source.

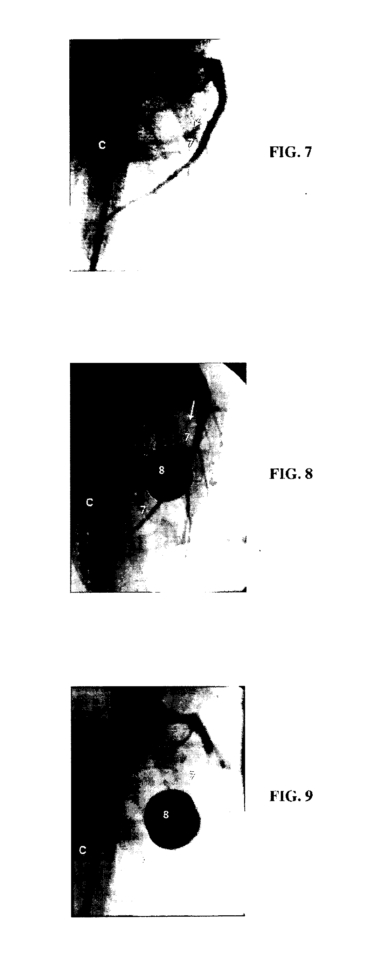

Figures 7-9: Evaluation of MMD on the coronary artery of a farm pig.
   Fig. 7: A baseline angiogram of the left anterior descending coronary artery of a farm pig.
   Fig. 8: An angiogram of the left anterior descending coronary artery with the external magnetic field source placed on the ribs.
   Fig. 9: Complete occlusion of the left anterior descending coronary artery is shown after two minutes of infusion of the microparticle dispersion.

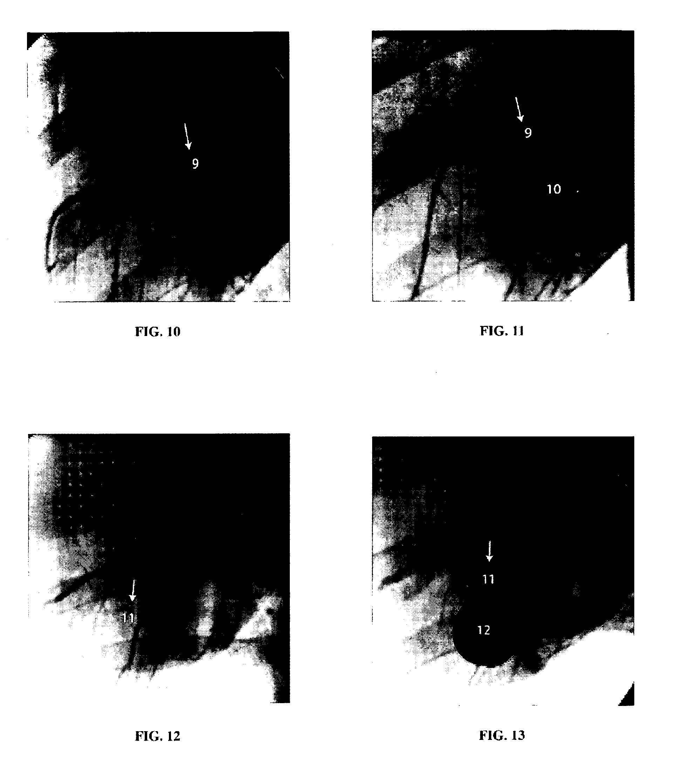

Figures 10-13: Subselection of coronary branches of farm pigs and the respective placement of the EMFS for the desired site of occlusion.
   Fig. 10: A baseline angiogram of the left coronary artery.
   Fig. 11: An angiogram of the left coronary artery with the EMFS placed selectively above the rumus branch on the ribs.
   Fig. 12: Another baseline angiogram of the left coronary artery.
   Fig. 13: An angiogram of the left coronary artery with the EMFS placed selectively above the diagonal branch on the ribs.

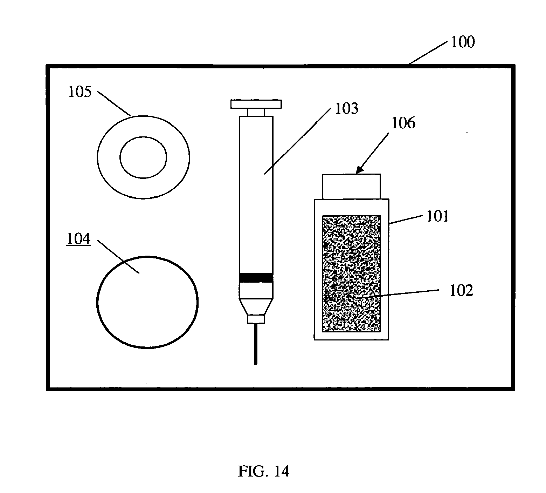

Figure 14: An example of a medical or surgical kit

### Detailed Description of the Invention

The present invention discloses a method for the occlusion of blood vessels or fluid vessels to stop the flow of blood or fluid, especially during elective or emergency surgical procedures or following traumatic injury. The present invention also encompasses a medical device, kit, clinical tool, or operating tool for occluding or controlling blood flow or fluid flow.

The method for occluding or controlling blood flow or fluid flow is comprised of the following steps:
(a) magnetic microparticles, magnetic nanoparticles, or magnetic colloids are administered in the form of a dispersion or suspension into a blood vessel or fluid vessel that flows through the vessel in the bloodstream or fluid flow, and
(b) an external magnetic field source is placed or held at the desired site near or above the path of the bloodstream or fluid flow, and
(c) a plug or blockade is formed within said blood vessel or fluid vessel as a result of acts (a) and (b) that occludes or controls blood flow or fluid flow through the vessel.

The medical device, kit, clinical tool, or operating tool for occluding or controlling blood flow or fluid flow is comprised of:
(a) magnetic microparticles, magnetic nanoparticles, or magnetic colloids in a container which can be administered in the form of a dispersion or suspension into a blood vessel or fluid vessel that flows through the vessel in the bloodstream or fluid flow, and
(b) an external magnetic field source which is adapted to be placed or held at the desired site near or above the path of the bloodstream or fluid flow to form a plug or blockade within said blood vessel or fluid vessel that occludes or controls blood flow or fluid flow, and
(c) an outer container enclosing said external magnetic field source and said container containing the magnetic microparticles, magnetic nanoparticles, or magnetic colloids.

The medical device, kit, clinical tool, or operating tool which also encompasses the method (or vice versa) for occluding or controlling blood flow or fluid flow are hereinafter grouped under the term "Magnetic Medical Device" or "MMD". The magnetic microparticles, magnetic nanoparticles, and/or magnetic colloids are hereinafter grouped under the term "magnetic microparticles". The dispersion, suspension, or solution of the magnetic microparticles, magnetic nanoparticles, or magnetic colloids are hereinafter grouped under the term "dispersion". The external magnetic field source to be placed or held at the desired site near or above the path of the blood stream or fluid flow is hereinafter referred to as "external magnetic field source" or "EMFS".

In the principal embodiment of the present invention, a plug or blockade is formed within the blood vessel or fluid vessel to occlude or control blood flow or fluid flow when (a) a dispersion of magnetic microparticles is introduced into a blood vessel or fluid vessel, and (b) an external magnetic field source is placed or held at the desired site near or above the path of the bloodstream or fluid flow.

This novel technology can be applied for the occlusion of blood vessels to stop the flow of blood during elective or emergency surgical procedures or following traumatic injury. Vascular blood flow in a blood vessel is now most commonly occluded during surgery by either clamping or ligation. Traditional clamping or ligation results in trauma to the vessel and disruption of the endothelial cell permeability barrier as described above. The present invention is a better clamping method or device to occlude vascular blood flow in a blood vessel during surgery. This is because the magnetic microparticles in the occluding plug exert very little pressure or shear force on the endothelial cell lining. This novel technology can be applied for the occlusion of blood vessels to control internal hemorrhage. This new invention will offer the surgeon or paramedic a means of occluding blood vessels while causing little or no injury to the vessel itself, as compared with current methods of clamping or tying to occlude blood flow, which damage both the endothelial cells lining the vessel as well as the smooth muscle cells in the vessel wall.

The MMD for occluding or controlling blood flow or fluid flow in this invention is quite flexible. It can be used for any application involving occlusion, reduction, or control of blood flow or fluid flow. The invention can be adapted for a wide range of surgical and medical procedures. The potential applications can be extended to virtually every aspect of the surgical and trauma treatment fields. The MMD for occluding or controlling blood flow or fluid flow in this invention can be adapted or packaged in the form of a medical device, kit, clinical tool, or operating tool to suit the application. The medical device, kit, clinical tool, or operating tool can include magnetic microparticles, external magnetic field source, syringe, catheter, and other accessories. The magnetic microparticles can be prepared sterile under aseptic conditions or sterilized by γ-radiation, heat, chemicals or combination thereof.

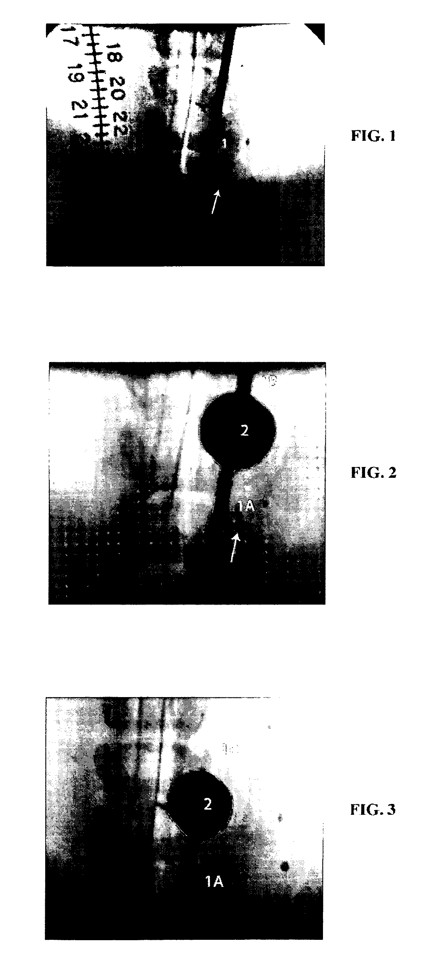

In this invention, a plug or blockade is formed to occlude or control blood flow or fluid flow when magnetic microparticles are administered into a blood vessel or fluid vessel, and an external magnetic field source is placed or held at the desired site near or above the path of the bloodstream or fluid flow. The EMFS can be placed or held at the desired site near or above the path of the blood stream or fluid flow either before, during, or after the infusion of the magnetic microparticles into the blood vessel or fluid vessel. An EMFS, such as a powerful NdFeB magnet no larger than an inch in diameter and weighing no more than five grams, can be placed near or above the vessel to be occluded for no more than the time required to maintain the blockade/occlusion. The design and utilization of this technology are demonstrated in Examples and Figures 1-13. It is important to point out that the magnetic microparticle preparations tested in vivo were indeed able to form a plug, under physiological flow conditions, in all three artery types tested (carotid, femoral, and coronary; see Examples and Figures 1-9). This phenomenon is rapid and completely reversible. The effects of plug formation can be either non-reversible or reversible. After the purpose of the occlusion is complete, the EMFS can be removed to dissolve or redisperse the microparticle plug or blockade resulting in the resumption of blood flow. This is shown in Examples and Figures 4-6. Therefore, after the required occlusion, there is no need for surgical removal because the microparticles redisperse into a colloidal form. The magnetic microparticles are also biodegradable and non-toxic. The required external magnetic field source can be placed or held either internal or external to the body, even though the external magnetic field source is external to the particular blood vessel or other vessel of interest. Non-invasive, minimally invasive, or invasive procedures can be utilized with the MMDs.

It has been shown in the inventor's laboratory that various dispersions of magnetic microparticles, under flow conditions, display rapid formation of a plug in the presence of an external magnetic field. As soon as the external magnetic field source is removed, the redispersion or dissolution of the plug occurs, allowing the resumption of flow inside the blocked vessel.

*In vitro* plug formation was observed to occur as soon as the particles were injected with the EMFS in place. In other words, the moment the particles passed under the EMFS, a plug was formed to block flow. Plug formation *in vivo* was also rapid. The limitation on determining how rapid was due to the time required to inject contrast media and take the x-ray. Thus, blockage occurred within 30 seconds to 2 minutes.

Concerning plug dispersal, in vitro experiments indicated that this occurred rapidly, i.e., within 30 to 90 seconds. Complete plug dispersal *in vivo* was slower than *in vitro*, taking between three and eight minutes in the femoral artery (Fig. 5).

Magnetic microparticles with a range of diameters, surface properties, magnetic properties, and microencapsulating materials were used in the inventor's laboratory for the occlusion of blood flow or fluid flow. The plug formation of the microparticle dispersion in the presence of an EMFS, and the redispersion of the microparticles after the removal of the external magnetic field depend on particle size and size distribution, surface characteristics, magnetic properties, and matrix properties.

In order to occlude specific blood vessels, sizes were chosen from a range with an upper limit dictated by the diameter of the venules, vessels, or capillaries and a lower limit set by the ability to form a useful plug. One would not want the size to be so big that there is occlusion in the absence of an EMFS. The sizes of the magnetic microparticles ranges from 0.01 to 2000 micrometers, preferably 0.01 to 200 micrometers, and optimally 0.02 to 50 micrometers.

The magnetic microparticles are comprised of any magnetizable materials such as paramagnetic, superparamagnetic, ferrimagnetic, or ferromagnetic materials. The magnetic microparticles are alternatively comprised of one or a plurality of plain magnetic materials, surface-modified materials, surface-treated materials, coated microparticles, coated nanoparticles, microencapsulated microparticles, microencapsulated nanoparticles, microencapsulated microspheres, microencapsulated colloidal systems, microencapsulated liposomes and emulsions, or embedded systems comprised of magnetic ingredients.

The magnetic microparticles are alternatively comprised of or treated with emulsifiers, surfactants, lipids, polymers, copolymers, silicones, silanes, or other agents which modify the surface or encapsulate. The magnetic microparticles are alternatively comprised of, or additionally incorporated with, one or a plurality of therapeutic drugs, pharmaceutical compounds, hemostatic agents, or other bioactive agents to enhance the performance of the therapy or treatment.

The external magnetic field source is comprised of, but not limited to: (a) rare earth magnets including, but not limited to, Neodymium Iron Boron magnets, bonded Neodymium Iron Boron magnets, and Samarium Cobalt magnets, or (b) ceramic magnets, or (c) ceramic ferrite magnets, or (d) Alnico magnets, or (e) any other permanent magnets. The external magnetic field source is alternatively comprised of electromagnets, superconducting magnets, or any other magnetic field generators.

EMFS no larger than an inch in diameter, weighing no more than five grams, and comprised of powerful Neodymium-Iron-Boron (NdFeB) magnets, were used in the animal studies. With energy products ranging from 26 to 48 MGOe, NdFeB is the preferred choice for these high performance applications. The EMFS can be tailor-made to the size and shape (contoured to the needs of the surgeon) and required attraction for clinical studies. This treatment will be easy to reduce to practice in the clinical setting with a NdFeB magnet of the size of a quarter (one inch diameter) and weighing about five grams. The magnetic microparticles and the external magnetic field source can be customized to suit the application and adapted or packaged in various formats such as a medical device, surgical kit, operating kit, clinical tool, or operating tool. The medical device, surgical kit, operating kit, clinical tool, or operating tool can also include a syringe, catheter, and other accessories, and the magnetic microparticles, syringe, and catheter can be sterile.

### Examples

Magnetic microparticles with various diameters and magnetic properties were developed, tested and evaluated in animal models for their efficiency to occlude blood flow in various vessels.
a) Fine dispersions of magnetizable materials such as magnetite were prepared using procedures well-established at the inventors facility and briefly described as follows: Fine dispersions of magnetite were prepared by dissolving divalent Fe ions and trivalent Fe ions in an aqueous solution and adding an alkali metal hydroxide to the solution with thorough mixing to form fine particles of magnetite. The diameters of the above magnetic microparticles ranged from 0.01 to 50 micrometers. These microparticles are superparamagnetic and their attraction time on a small linchx2inch ceramic magnet is 1-2 seconds. These magnetic particles are highly hydrophilic. Magnetic susceptibilities of these magnetic microparticles are 30000-40000 cgs/g.
b) Surface modified magnetic microparticles were prepared by treating the above magnetizable materials with emulsifiers, surfactants, lipids, or other reagents. The following surface-active materials were used: long-chain carboxylates, alkyl sulfonates, oxyethylenated nonyl phenols, polyvinyl alcohols, polyethylene glycol, and silanes. Surface-modified particles include charged surface groups or moieties for better or longer circulation in the blood, thereby allowing them to recirculate and to be trapped at the desired site using an external magnetic field. The diameters of the above magnetic microparticles ranged from 0.01 to 100 micrometers. These microparticles are superparamagnetic and their attraction time on a small linchx2inch ceramic magnet is 1-2 seconds. Magnetic susceptibilities of these magnetic microparticles are 30000-40000 cgs/g.
c) Polymer coated or encapsulated magnetic microparticles were prepared using the above magnetizable materials and polymer materials such as polylactides and copolymers of lactide and glycolide. These microparticles are super paramagnetic and their attraction time on a small linchx2inch ceramic magnet is 1-3 seconds. Magnetic susceptibilities of these magnetic microparticles are 30000-50000 cgs/g. The diameters of the above magnetic microparticles ranged from 0.01 to 200 micrometers. Polymer encapsulated magnetic microparticle systems are additionally incorporated with therapeutic drugs, pharmaceutical compounds, hemostatic agents, or other bioactive agents.

The external magnetic field sources suited for these applications include Neodymium-Iron-Boron (NdFeB) magnets. In these experiments, NdFeB disc magnets were used.

### In Vivo Studies on MMDs for Occlusion of Blood Flow

The MMDs have been tested and evaluated in animal models for their efficiency to occlude blood flow in various vessels. Animal studies have been performed to show the technology for *in vivo* use. Examples of experiments conducted are shown below and in Figures 1-13. The Figures 1-13 are black and white x-ray type photographs of the original angiograms. Figures 1-13 are angiograms showing vessels during different time periods in the method. The advantageous effects of the invention occur entirely underneath the skin. The most important point is that the angiogram photographs are genuine proof that the invention operates successfully.

Using juvenile farm pigs as an experimental model, the MMDs have been tested for their efficiency to occlude blood flow in various vessels. It has been shown that the new MMDs can occlude the blood flow in carotid, femoral, and coronary arteries.

Experimental Protocol: Juvenile farm pigs weighing 50 to 60 kg were used in this study. The animals were divided into 3 groups: carotid artery, femoral artery, and coronary artery. All animals received a normal diet. Animals underwent either femoral or carotid artery cut down. An 8F sheath was inserted into the artery. After systemic heparinization, an 8F guiding catheter was used to engage carotid, femoral, or coronary arteries. After a baseline arterial angiogram was performed, the EMFS was placed over the target artery. Contrast material was injected to insure that the target vessel was not in spasm and the EMFS was positioned over the target vessel. As soon as the position of the EMFS was optimized, the dispersed magnetic microparticles were infused at a rate of 1 ml/min. Angiography was performed repeatedly 30-60 seconds after the infusion of microparticles to assess arterial flow.

Results: Local delivery of the microspheres was well-tolerated by all animals. No hemodynamic, heart rate, or blood pressure changes were noted. Successful occlusion of blood flow by the medical device was achieved in carotid, femoral, and coronary artery beds.

An example of such successful occlusion of blood flow with the MMD in the carotid artery is shown in Figures 1-3. Figure 1 is the baseline angiogram of a farm pig's right carotid artery (1) with the arrow showing the direction of the blood flow. Figure 2 is an angiogram with an external magnetic field source (2) placed on the skin above the pig's right carotid artery; continuous blood flow is evident on either side of the external magnetic field source (2) in the carotid artery (1A) and (1 B). Figure 3 is an angiogram showing the pig's occluded right carotid artery one minute after microparticle infusion. Blood flow in the carotid artery (1A) is stopped right at the point below the external magnetic field source (2) and no blood flow is seen in the carotid artery (1 B) beyond the external magnetic field source (2), which indicates a complete occlusion of blood flow in the right carotid artery.

An example of a successful occlusion of blood flow with the MMD in the femoral artery and resumption of blood flow on removal of the external magnetic field source is shown in Figures 4-6. Figure 4 is an angiogram showing "no blood flow" in the superficial femoral artery (3) of a farm pig less than one minute after microparticle infusion with an external magnetic field source (4) placed on the skin above the pig's superficial femoral artery (3). Thus selective total occlusion of the superficial femoral artery (3) is achieved. Figure 5 is an angiogram showing a partial resumption of blood flow in the superficial femoral artery (3) three minutes after removal of the external magnetic field source. Figure 6 is an angiogram showing a complete resumption of the blood flow in the superficial femoral artery (3) eight minutes after removal of the external magnetic field source. The phenomenon is therefore completely reversible.

An example of a successful occlusion of blood flow with the MMD in the coronary artery is shown in Figures 7-9. Figure 7 is a baseline angiogram of the left anterior descending coronary artery (7) of a farm pig. In Figures 7-9, the guiding catheter (C) is used to engage the coronary arteries. Figure 8 is an angiogram of the left anterior descending coronary artery (7) with the external magnetic field source (8) placed on the ribs. Figure 9 is an angiogram showing "no blood flow" in the left anterior descending coronary artery (7) two minutes after infusion of the microparticle dispersion. A complete occlusion of blood flow in the left anterior descending coronary artery is achieved.

Occlusion was rapid in all cases. The target artery resumed blood flow after removal of the EMFS within minutes. This is shown in the example with the femoral artery in Figures 4-6. These results clearly demonstrate the usability of the magnetic microparticles in conjunction with the EMFS as a MMD to block blood flow reversibly *in vivo*. The MMD can be adapted to any branches of the arteries. Figures 10-13 show the subselection of coronary branches and the respective placement of the EMFS for the desired site of occlusion. Figure 10 is a baseline angiogram of the left coronary artery of a farm pig. Figure 11 is an angiogram of the left coronary artery with the external magnetic field source (10) placed selectively above the rumus branch (9) on the ribs. Figure 12 is another baseline angiogram of the the left coronary artery of a farm pig. Figure 13 is an angiogram of the left coronary artery with the external magnetic field source (12) placed selectively above the diagonal branch (11) on the ribs. The coronary branches shown can be selectively occluded for various applications.

Safe and effective reversible occlusion of vascular blood flow has been demonstrated in animals under surgical conditions. A range of products in kit or device form could be used in a variety of surgical and trauma applications. The kit or device form can include magnetic microparticles, EMFS, syringe, catheter, and other accessories, and the magnetic microparticles, syringe, and catheter can be sterile. Preferably, the kit or device, an example of which is shown in Figure 14, includes an outer container 100 (typically in the form of a covered tray, a pouch, or box), magnetic microparticles 102 either in a vial, bottle, or other container 101 (and can be sterile therein), and a syringe 103 with (or without) a needle, or other medical device (such as a catheter) to allow infusion of the magnetic microparticles into a selected blood vessel or fluid vessel. More preferably, the sterile magnetic microparticles are preloaded and already in the syringe in the kit. It this case, a separate vial 101 is not required, but optionally may be included as well. Also, the kit may include two or more such vials containing the magnetic microparticles and/or two or more such syringes, with or without being preloaded with magnetic microparticles. The kit also includes a magnet or EMFS 104. Optionally, EMFS 104 can include an adhesive on one or more faces thereof, with an optional peel-away facing to expose the adhesive, with the adhesive helping to keep the EMFS in position on the patient, such as on their skin at the proper location. Also optionally, tape 105 or other such holding device (such as elastic banding or Velcro) may be included in the kit to aid in holding the EMFS in position. The kit may also include instructions and warnings included in a paper insert. Optionally, but preferably, in the device and/or kit, the container (such as a vial, bottle, bag, or otherwise) may include a penatratable member, such as an elastomeric, foil, wax, paper or other such member 106 or cap to allow penetration by a needle there through for withdrawal of the magnetic particles from the sterile container.

A method is provided wherein the magnetic microparticles are first prepared or made, then medically sterilized by heat, radiation (gamma or otherwise), chemicals or otherwise, then placed in a sterile container (such as a vial, bottle, or syringe, for example). Also, the particles may be prepared or made aseptically, avoiding the need for subsequent sterilization. Thereafter, the container may be labeled, packaged, and/or placed as part of a kit (described above).

### Applications of MMD

The new MMDs offer the surgeon or paramedic a means of occluding blood vessels while causing little or no injury to the vessel itself, as compared with current methods of clamping or tying to occlude blood flow, which damage both the endothelial cells lining the vessel as well as the smooth muscle cells in the vessel wall. This is because the magnetic microparticles in the occluding plug exert very little pressure or shear force on the endothelial cell lining. An EMFS can be placed at the desired site of occlusion near or above the vessel to be occluded for no more than the time required to maintain the blockade/occlusion. The MMD can be used during surgery to arrest blood flow in a reversible manner without the use of clamps or ligatures. The MMD could be used for the occlusion of blood vessels to stop the flow of blood during elective or emergency surgical procedures or following traumatic injury. The MMD could be adapted for a wide range of surgical and other medical procedures, including, but not limited to cardiac bypass surgery, vascular surgery, renal transplantation, hemorrhage control, EMT trauma, and battlefield trauma.

### Examples of Applications

This new technology could be utilized during surgical or medical procedures involving the coronary artery. Figures 7-13 show the usability of the MMD on the coronary artery of a farm pig. Figure 8 shows the baseline angiogram of the left anterior descending coronary artery with the external magnetic field source placed on the ribs. Figure 9 shows a complete occlusion of blood flow in the left anterior descending coronary artery after microparticle infusion.
1. Coronary artery bypass grafting (CABG): The use of the MMD to occlude blood flow in the left anterior descending coronary artery (LAD) during bypass grafting would avoid any compression of the coronary artery during the procedure, and spare the endothelial cell lining from injury. This should improve the long-term surgical outcome by minimizing atherosclerotic changes in the occluded region of the vessel.
2. Surgical correction of deep vein thrombosis (DVT): The use of the MMD to occlude blood flow in the affected vein in lieu of clamping or tying would avoid damaging the vein in that region and prevent the introduction of a possible new attachment site for post surgical thrombus formation.
3. For controlling hemorrhage: The MMD is capable of controlling hemorrhage from vascular injuries in the proximal extremities. The device will be especially useful for the treatment of injuries that are not amenable to tourniquet application. Vascular injuries in the region of the groin continue to be largely untreatable. The MMD technology can easily be used to control hemorrhage in the groin. The usability of the device for this application is shown in the example with the femoral artery of a farm pig (Figures 4-6). Figure 4 shows selective total occlusion of blood flow in one of the superficial femoral arteries after microparticle infusion. This process can also be reversible, in that after the necessary procedure, blood flow can be resumed. Figure 6 shows complete resumption of the blood flow after removal of the external magnetic field source.
   The MMD could also be used to control noncompressible hemorrhage. Noncompressible hemorrhage continues to be a primary cause of death in both military and civilian trauma (Bellamy RF, Mil. Med. 149:55-62, 1984; Sauaia A et al, J. Trauma 38:185-193, 1995). Currently there is no treatment short of surgery for severe abdominal bleeding resulting from either blunt or penetrating injury. Utilization of the new device, that is by infusion of magnetic microparticles through a trocar and holding the external magnetic field source above the site, will stop the bleeding. A hemostatic agent could be included in the magnetic microparticle dispersion to promote coagulation and provide hemostasis. The MMD can also control severe intracavitary hemorrhage. The MMD can limit the immediate, short- and long-term deleterious consequences of severe hemorrhage. This procedure can be noninvasive, minimally invasive, or invasive.
4. Other applications:
   The examples outlined above as possible applications for the occlusion of blood flow by the use of the MMD are intended only to be suggestive of the many possible uses of the MMD in surgical and other fields. The area of trauma surgery, including battlefield trauma, and the use of the MMD by paramedics to stop hemorrhage, are two other medical applications that could prove effective.
   Future products could include surgical kits to be used in cardiac bypass surgery, vascular surgery, renal transplant surgery, EMT trauma, and battlefield trauma. Future products could include kits for embolization. In addition, future products could include site-specific drug delivery.
   The MMD for occluding or controlling blood flow or fluid flow in this invention is quite flexible. It can be used for any application involving occlusion, reduction, or control of blood flow or fluid flow. The invention can be adapted for a wide range of applications. The potential applications can be extended to virtually every aspect of the medical, surgical, clinical, and other fields. The MMD for occluding or controlling blood flow or fluid flow in this invention can be adapted or packaged in the form of a medical device, kit, clinical tool, or operating tool to suit the application. The medical device, kit, clinical tool, or operating tool can include magnetic microparticles, external magnetic field source, syringe, catheter, and other accessories. The magnetic microparticles, syringe, and catheter can be sterile.

## Claims

1. A medical or surgical kit for occluding or controlling blood flow or fluid flow comprised of:
(a) magnetic microparticles, magnetic nanoparticles, or magnetic colloids in a container which can be administered in the form of a dispersion or suspension into a blood vessel or fluid vessel that flows through the vessel in the bloodstream or fluid flow, and
(b) an external magnetic field source which is adapted to be placed or held at the desired site near or above the path of the bloodstream or fluid flow to form a plug or blockade within said blood vessel or fluid vessel that occludes or controls blood flow or fluid flow.

2. The medical or surgical kit for occluding or controlling blood flow or fluid flow of any preceding claim wherein the sizes of said magnetic microparticles, magnetic nanoparticles, or magnetic colloids are in the size range of 0.01 µM to 2000 µM, with
(a) the maximum size of said magnetic microparticles, magnetic nanoparticles, or magnetic colloids being chosen such that they are smaller than the diameter of the venules, vessels, or capillaries, and
(b) the maximum size of said magnetic microparticles, magnetic nanoparticles, or magnetic colloids being chosen such that they do not independently occlude blood flow or fluid flow without the applied external magnetic field.

3. The medical or surgical kit for occluding or controlling blood flow or fluid flow of any preceding claim wherein said magnetic microparticles, magnetic nanoparticles, or magnetic colloids are comprised of any magnetizable materials including, but not limited to, paramagnetic, superparamagnetic, ferrimagnetic, and ferromagnetic materials.

4. The medical or surgical kit for occluding or controlling blood flow or fluid flow of any preceding claim wherein said magnetic microparticles, magnetic nanoparticles, or magnetic colloids are comprised of plain magnetic materials, surface-modified materials, surface-treated materials, coated microparticles, coated nanoparticles, microencapsulated microparticles, microencapsulated nanoparticles, microencapsulated microspheres, microencapsulated colloidal systems, microencapsulated liposomes and emulsions, or embedded systems comprised of magnetic ingredients, or magnetic systems comprised of additionally incorporated drugs, pharmaceutical compounds, hemostatic agents or other bioactive agents.

5. The medical or surgical kit for occluding or controlling blood flow or fluid flow of any preceding claim wherein said external magnetic field source is comprised of:
(a) rare earth magnets including, but not limited to, Neodymium Iron Boron magnets, bonded Neodymium Iron Boron magnets, and Samarium Cobalt magnets, or
(b) ceramic magnets, or
(c) ceramic ferrite magnets, or
(d) Alnico magnets, or
(e) electromagnets, or
(f) superconducting magnets.

6. The medical or surgical kit for occluding or controlling blood flow or fluid flow of any preceding claim when used for any surgical application or medical application involving occlusion or control of blood flow or fluid flow in humans or in animals.

7. The medical or surgical kit for occluding or controlling blood flow or fluid flow of any preceding claim when used for controlling or managing blood leaks or fluid leaks, and further comprised of an outer container enclosing said external magnetic field source and said container containing the magnetic microparticles, magnetic nanoparticles, or magnetic colloids.

8. The medical or surgical kit for occluding or controlling blood flow or fluid flow of any preceding claim wherein said magnetic microparticles, magnetic nanoparticles, or magnetic colloids are sterile.

9. The medical or surgical kit for occluding or controlling blood flow or fluid flow of any preceding claim wherein said container containing the magnetic microparticles, magnetic nanoparticles, or magnetic colloids is a vial or bottle, and wherein said container further includes a penetratable member or cap to allow penetration by a needle there through for withdrawal of the magnetic particles from the sterile container.

10. The medical or surgical kit for occluding or controlling blood flow or fluid flow of any preceding claim wherein said container containing the magnetic microparticles, magnetic nanoparticles, or magnetic colloids is a syringe with or without a needle.

11. The medical or surgical kit for occluding or controlling blood flow or fluid flow of any preceding claim further includes a syringe and optionally a catheter for infusion, and separate containers containing said magnetic microparticles, magnetic nanoparticles, or magnetic colloids.

12. The medical or surgical kit for occluding or controlling blood flow or fluid flow of any preceding claim further includes an adhesive for adhering said external magnetic field source in position.

13. A method for occluding or controlling blood flow or fluid flow wherein:
(a) magnetic microparticles, magnetic nanoparticles, or magnetic colloids are administered in the form of a dispersion or suspension into a blood vessel or fluid vessel that flows through the vessel in the bloodstream or fluid flow, and
(b) an external magnetic field source is placed or held at the desired site near or above the path of the bloodstream or fluid flow, and
(c) a plug or blockade is formed within said blood vessel or fluid vessel as a result of acts (a) and (b) that occludes or controls blood flow or fluid flow through the vessel.

14. The method for occluding or controlling blood flow or fluid flow of claim 13 wherein the sizes of said magnetic microparticles, magnetic nanoparticles, or magnetic colloids are in the size range of 0.01 µM to 2000 µM, with
(a) the maximum size of said magnetic microparticles, magnetic nanoparticles, or magnetic colloids being chosen such that they are smaller than the diameter of the venules, vessels, or capillaries, and
(b) the maximum size of said magnetic microparticles, magnetic nanoparticles, or magnetic colloids being chosen such that they do not independently occlude blood flow or fluid flow without the applied external magnetic field.

15. The method for occluding or controlling blood flow or fluid flow of claim 13 wherein said magnetic microparticles, magnetic nanoparticles, or magnetic colloids are comprised of any magnetizable materials including, but not limited to, paramagnetic, superparamagnetic, ferrimagnetic, and ferromagnetic materials.

16. The method for occluding or controlling blood flow or fluid flow of claim 13 wherein said magnetic microparticles, magnetic nanoparticles, or magnetic colloids are comprised of plain magnetic materials, surface-modified materials, surface-treated materials, coated microparticles, coated nanoparticles, microencapsulated microparticles, microencapsulated nanoparticles, microencapsulated microspheres, microencapsulated colloidal systems, microencapsulated liposomes and emulsions, or embedded systems comprised of magnetic ingredients, or magnetic systems comprised of additionally incorporated drugs, pharmaceutical compounds, hemostatic agents or other bioactive agents.

17. The method for occluding or controlling blood flow or fluid flow of claim 13 wherein said external magnetic field source, when applied to humans or to animals, is either internal or external to the body, even though the said external magnetic field source is external to the particular blood vessel or vessel of interest.

18. The method for occluding or controlling blood flow or fluid flow of claim 13 wherein said external magnetic field source is comprised of:
(a) rare earth magnets including, but not limited to, Neodymium Iron Boron magnets, bonded Neodymium Iron Boron magnets, and Samarium Cobalt magnets, or
(b) ceramic magnets, or
(c) ceramic ferrite magnets, or
(d) Alnico magnets, or
(e) electromagnets, or
(f) superconducting magnets.

19. The method for occluding or controlling blood flow or fluid flow of claim 13 having
(a) reversible effects upon removal of said external magnetic field source, or
(b) irreversible effects upon removal of said external magnetic field source.

20. The method for occluding or controlling blood flow or fluid flow of claim 13 when used for any surgical application or medical application involving occlusion or control of blood flow or fluid flow in humans or in animals.

21. The method for occluding or controlling blood flow or fluid flow of claim 13 when used for controlling or managing blood leaks or fluid leaks.

22. A device comprised of:
(a) a sterile container, and,
(b) sterile magnetic microparticles, magnetic nanoparticles, or magnetic colloids in said container which can be administered in the form of a dispersion or suspension into a blood vessel or fluid vessel that flows through the vessel in the bloodstream or fluid flow.

23. The device of claim 22 wherein the sizes of said magnetic microparticles, magnetic nanoparticles, or magnetic colloids are in the size range of 0.01 µM to 2000 µM, with
(a) the maximum size of said magnetic microparticles, magnetic nanoparticles, or magnetic colloids being chosen such that they are smaller than the diameter of the venules, vessels, or capillaries, and
(b) the maximum size of said magnetic microparticles, magnetic nanoparticles, or magnetic colloids being chosen such that they do not independently occlude blood flow or fluid flow without the applied external magnetic field.

24. The device of claim 23 wherein said sterile container is a syringe.

25. The device of claim 23 wherein said sterile container is a vial or bottle.

26. The device of any preceding claim wherein said container includes a penetratable member or cap to allow penetration by a needle there through for withdrawal of the magnetic particles from the sterile container.
